Europäisches Patentamt

European Patent Office

Office européen des brevets

(11) Publication number: **0 233 937 B1**

## EUROPEAN PATENT SPECIFICATION

(45) Date of publication of patent specification: **13.01.93**  (51) Int. Cl.5: **C12N  1/16**

(21) Application number: **86905524.4**

(22) Date of filing: **15.08.86**

(86) International application number:
**PCT/US86/01702**

(87) International publication number:
**WO 87/01129 (26.02.87 87/05)**

(54) FERMENTATION METHODS FOR HEPATITIS VACCINE PRODUCTION.

(30) Priority: **15.08.85 US 766054**

(43) Date of publication of application:
**02.09.87 Bulletin  87/36**

(45) Publication of the grant of the patent:
**13.01.93 Bulletin  93/02**

(84) Designated Contracting States:
**AT BE CH DE FR GB IT LI LU NL SE**

(56) References cited:
**EP-A- 72 318          EP-A- 0 078 500**
**EP-A- 0 157 275      EP-A- 0 165 613**
**EP-A- 0 171 000      EP-A- 0 219 791**
**US-A- 4 167 450**

**CHEMICAL ABSTRACTS, vol. 92, no. 11,
March 1980, page 457, abstract no. 92718r,
Columbus, Ohio, US; & JP-A-79 129 181
(NIPPON BEET SUGAR MFG. CO. LTD)
06-10-1979**

(73) Proprietor: **AMGEN INC.
1840 Dehavilland Drive
Thousand Oaks California 91320 -1789(US)**

(72) Inventor: **FIESCHKO, John, C.
711 Flaming Star Drive
Thousand Oaks, CA 91360(US)**

(74) Representative: **Brown, John David et al
FORRESTER & BOEHMERT Franz-
Joseph-Strasse 38
W-8000 München 40(DE)**

Rank Xerox (UK) Business Services

CHEMICAL ABSTRACTS, vol. 88, no. 13, March 1978, page 369, abstract no. 87648f, Columbus, Ohio, US; & JP-A-77 125 686 (MARUBISHI RIKA SOCHI KENKYUSHO K.K.; ORIENTAL YEAST CO., LTD) 21-10-1977

CHEMICAL ABSTRACTS, vol. 100, no. 15, 9th April 1984, page 447, abstract no. 119314d, Columbus, Ohio, US; T. SUMITANI et al.: "Automatic control of fermentor using microcomputer", & BIOTECH 83 [EIGHTY-THREE], PROC. INT. CONF. COMMER. APPL. IMPLIC. BIOTECHNOL., 1ST 1983, 295-306

DSM (Deutsche Sammlung von Mikororganismen), Strain catalogue, 1983, p. 271.

GENE (Amsterdam, NL) volume 32, issued November 1984 (Bitter et al) "Expression of heterologous genes in Saccharomyces cerevisiae from vectors utilizing the glyceraldehyde-3-phosphate dehydrogenase gene promoter", see pages 263-265

Biotechnology and Bioengineering (New York) volume 23 issued 1981 (Woehrer et al) "Regulatory aspects of Baker's yeast metabolism in aerobic fed-batch cultures", see pages 569-580

**Description**

Background

The present invention relates in general to methods and media for the growth of yeast strains expressing Hepatitis B surface Antigen (HBsAg) and in particular to methods and media for the growth of yeast strains expressing HBsAg under aerobic, glucose-limited conditions, relatively low temperatures and relatively high levels of free amino acids.

The hepatitis B virus causes a disease now known as hepatitis B, but formerly known as "serum hepatitis". It has been estimated that there are more than 200,000,000 people who persistently have hepatitis B virus in their blood. Infection with the virus is a major cause of acute liver disease. Carriers of hepatitis B virus have a high risk of contracting cirrhosis and hepatocellular carcinoma.

The human hepatitis B virus has been identified with the Dane particle which is found in the serum of carriers and which is the causative agent of clinical hepatitis B infection. The Dane particle is a 42 nanometer membrane structure which includes lipids, DNA, and at least four proteins: hepatitis B surface antigen (HBsAg), hepatitis B core antigen (HBcAg), hepatitis B e antigen (HBeAg), and a DNA polymerase. In their serum, carriers also have 22 nanometer lipid particles which contain HBsAg but not DNA, HBcAg, HBeAg or DNA polymerase. Hepatitis B vaccines currently in use employ 22 nanometer particles obtained from human plasma.

Human plasma used in the manufacture of hepatitis B vaccine has an antigen concentration of about 400 micrograms per milliliter. Because the total serum concentration is about 60 milligrams per liter, only a 150-fold purification is required. Wampler, et al., in "Modern Approaches to Vaccines," Chanock, et al., eds., Cold Spring Harbor Laboratory, Cold Spring Harbor, New York, pp. 251-256 (1984). However, inasmuch as the starting material is human plasma, plasma-derived hepatitis B vaccines are limited in supply and extreme caution must be exercised to insure that they are free of all harmful contaminating material, including infectious viruses.

Another approach to obtaining hepatitis B vaccine involves infecting cultured malignant cells, specifically hepatoma cells, with hepatitis B virus. HBsAg is harvested from preparations of lysed hepatoma cells. Knowles, et al., PCT Application No. PCT/US81/00778. Although this approach eliminates the need for human plasma, the undesirability of using cancer cell products in vaccines, the need for extreme caution to avoid infectivity and the difficulties inherent in mammalian cell culture all limit the usefulness of this approach.

Monkey kidney cells transfected with recombinant plasmids containing the gene for HBsAg have been reported as liberating HBsAg by lysis or by secretion. Levinson, et al., EPO Application No. 73,656. Nevertheless, with the exception of the elimination of concerns regarding use of products of cancerous cells, production of hepatitis B vaccine from monkey kidney fibroblasts shares the drawbacks inherent in the production of hepatitis B vaccine from hepatoma cells.

In light of the problems with conventional vaccine production, it is desirable to have methods for preparation of HBsAg in isolation from other components of the hepatitis B virus. Production of HBsAg in microorganisms by the use of recombinant technology permits such isolated production.

In one approach to applying recombinant technology to HBsAg production, the gene coding for HBsAg may be inserted into a bacterial plasmid and may be amplified and expressed in several Escherichia coli host organisms. Rutter, et al., European Patent Application No. 020,251. However, it has been reported that this technique results in low yields because HBsAg is easily degraded within E. coli and that growth of E. coli is inhibited by HBsAg. Miyanohara, et al., European Patent Application No. 105,049. It has also been reported that certain bacterial cell components, for example lipopolysaccharides, are highly toxic to humans and pose purification problems and it has been reported that bacteria, being prokaryotes (i.e., members of the group of organisms which lack in nucleus) may provide inefficient translation of the genes of eukaryotes (i.e., members of the group of organisms which possess a nucleus) inasmuch as: bacteria cannot perform certain processes, such as splicing out of introns or proteolytic cleavage of precursor proteins; bacteria do not glycosylate, phosphorylate or methylate proteins, all of which are post-translational modifications which may be important for the immunogenicity of proteins; bacteria do not recognize the so-called signal peptide which is important for secretion of gene products in eukaryotes; and codon preference (i.e. the facility with which a particular sequence of nucleic acids coding for an amino acid constituent of a protein is expressed) may be different in prokaryotic and eukaryotic organisms. Hofschneider, et al., European Patent Application No. 105,141.

Another approach to expression of HBsAg in microorganisms, and one which overcomes almost all of the objections to the use of E. coli as a host, employs a yeast expression system. Such expression systems

3

have generally involved the use of bacterial-yeast shuttle vectors, which are plasmids having sequences permitting replication in both bacteria and yeast. Rutter, et al., European Patent Application No. 072,318; Hitzenman, et al., European Patent Application No. 073,657; Cabezon, et al., European Patent Application No. 106,828; and Bitter, et al., Gene, 32, 263-274 (1984). Yeast offers several advantages for the production of eukaryotic gene products: yeast are GRAS organisms generally recognized as safe; yeast is readily grown in culture in large quantities; the technology of yeast culture on a large scale is well understood; because yeast cells are eukaryotic, they contain processing machinery for glycosylation, phosphorylation and methylation; and yeast cells better tolerate the HBsAg protein. Rutter, et al., supra.; Hitzeman, et al., supra. Furthermore, despite concerns that yeast-produced HBsAg might have a lower immunogenic potential than the form produced in mammalian cells (Hofschneider, et al., supra.), yeast-derived particles have an $ED_{50}$ (a measure of the effective dose necessary to elicit an immune response) of 112 mg which is only slightly higher than the $ED_{50}$ of 98 mg for mammalian cell-derived particles. Burnette, et al., in "Modern Approaches to Vaccines", Chanock, et al., eds., Cold Spring Harbor Laboratory, Cold Spring Harbor, New York, 245-250 (1984).

An important commercial aspect of the production of HBsAg polypeptides in yeast is the cost per gram. This cost is lowered by: (1) enhancing the growth of yeast; (2) by enhancing the production of or preventing the destruction of HBsAg polypeptides once produced.

In one approach to the growth of yeast, the culture is grown under glucose limitation whereby the glucose is maintained at a relatively low concentration, the growth rate is prevented from exceeding a given value and oxygen is maintained at a relatively high concentration. Using these techniques, the formation of ethanol is minimized. Fiechter et al., Advances in Microbial Physiology, 22, 123-183 (1981); in Yeast Technology, Reed et al, AVI Publishing Co., Inc., Westport, Connecticut (1973) pp. 53-81. For example, computer-controlled production of baker's yeast in a computer-coupled bioreactor may employ the respiratory quotient RQ (moles $CO_2$ produced/moles $O_2$ consumed) and the ethanol concentration as feedback parameters in automatic control of the rate of addition of glucose in a batch-fed medium. Woehrer et al., Biotechnol. Bioeng., 23, 567-581 (1981).

One type of yeast employed in the expression of recombinant gene products is Saccharomyces cerevisiae. See e.g., Bitter at al., Gene, 32, 263-274 (1984).

Components of media for the culture of yeast expressing recombinant gene products include: 0.2% w/v glucose in the minimal medium of Spizizen [Beggs, Nature, 275, 104-109 (1978; YEPD with 2% (w/v) peptone and 2% glucose, or 6.7 g/l of yeast nitrogen base, 5 g/l of casamino acids and 20 g/l of glucose for HBsAg expression [Rutter et al., European Patent Application No. 72,318]; YNB-leucine at 30 degrees C. for HBsAg expression [Hitzeman et al., European Patent Application No. 73657]; 0.67% yeast nitrogen base, 0.5% tryptophanfree casamino acids and 2% glucose for expression of HBsAg [Valenzuela et al., Nature, 298, 347-350 (1982)]; 2% glucose, 0.7% yeast nitrogen base amino acid and 2% YPD (2% polypeptone, 1% yeast extract and 2% glucose), or YPD alone or BurkHolder minimal medium at 30 degrees C. for expression of HBsAg [Miyanohara et al., European Patent Application No. 105,149]; 8.5 g/l yeast extract at ambient temperature (about 24 degrees C.) for the production of ethanol from xylulose [Gong et al., U.S. Patent No. 4,490,468]; 1% yeast extract, 2% peptone and 2% glucose for HBsAg expression [Valenzuela et al., Biotechnology, 3, 317-320 (1985)]; and yeast minimal salts medium plus 2% glucose or 10% hydrolyzed corn starch (with an average polymer size of 13 to 17 glucose molecules) or a continuous glucose feed for glucoamylase expression [Innis et al., Science, 228, 21-26 (1985)]. Such media are used for the batch growth of yeast and will typically result in both low cell concentrations and most likely low expression levels of HBsAg. For example, cells grown in YMS medium in shaker flasks typically give yields of only 50-200 ng/OD ml HBsAg. It is believed that a preferable medium and a preferable set of culture conditions remain to be defined for expressing foreign polypeptides in yeast.

## SUMMARY OF THE INVENTION

According to the present invention there is provided a medium for culturing yeast capable of expressing a foreign gene product comprising:

balanced free amino acids at a concentration between about 20 g/l and about 160 g/l.

The present invention provides a medium for culturing yeast capable of expressing a foreign gene product.

There is disclosed a medium comprising balanced free amino acids at a concentration between about 20 g/l and about 160 g/l.

According to the present invention there is further provided a method for the fermentation of yeast capable of expressing an exogenous gene product comprising the steps of:

introducing the yeast into a culture medium comprising balanced free amino acids at a concentration between about 20 g/l and about 160 g/l; and

culturing the yeast at a temperature of about 25 degrees C.

The present invention also provides a method for the growth of yeast capable of expressing a foreign gene product.

There is disclosed a method, wherein the yeast is introduced into a balanced medium including free amino acids at a concentration between about 20 g/l and about 160 g/l. The yeast is cultured at a temperature of about 25 degrees C.

Another method according to the present invention provides a method for the fermentation of yeast capable of expressing an exogenous gene product comprising the steps of:

culturing the yeast in a medium having carbon source concentration maintained above but as close to zero as possible and an ethanol concentration maintained as close to zero as possible; and

maintaining the temperature of the medium at about 25 degrees C. The yeast is cultured at a temperature of about 25 degrees C. and is fed a nutrient solution in such a way that the carbon source is maintained at a concentration above but as close to zero as possible and ethanol does not accumulate.

Brief Description of the Drawings

FIG. 1 illustrates a standard fermentation run for the fermentation of yeast expressing HBsAg;

FIG. 2 shows plots of ethanol concentration, glucose concentration and $OD_{600}$ for the run of FIG. 1;

FIG. 3 depicts growth yield data for the run of FIG. 1;

FIG. 4 is a graph of $OD_{600}$ for cells grown on minimal and rich media;

FIG. 5 is a plot of cell and ethanol concentration vs. time;

FIG. 6 illustrates the growth a a strain of yeast on ethanol with and without casamino acids;

FIG. 7 is a comparative plot of cell concentration vs. time at different concentrations of casamino acids;

FIG. 8 depicts growth profiles for a run on a medium supplemented with casamino acids

FIG. 9 illustrates growth profiles for a run on a medium supplemented with casamino acids

FIG. 10 illustrates growth profiles for a run on a medium according to the present invention;

FIG. 11 illustrates growth profiles for a run at reduced temperature according to the present invention;

FIG.12 illustrates a run on a minimal medium and at a reduced temperature according to the present invention; and

FIG. 13 depicts a run on a medium according to the present invention and at a reduced temperature according to the present invention.

Detailed Description

Efficient growth of S. cerevisiae to high cell concentrations on glucose has encountered two fundamental problems in the past: (1) the growth yield of the organism (g yeast dry weight/g glucose) is low and (2) the maximum cell concentration reached is typically lower than values of 30-80 g yeast dry weight/liter frequently reported in the literature.

An investigation was undertaken to account for all the carbon added to the fermentor in the form of glucose during a standard fermentation run to attempt to determine a carbon balance between substrate glucose and products of cells and carbon dioxide).

Example 1

A "standard" run of the sort normally used for production of hepatitis B surface antigen fermentation of S. cerevisiae was carried out. In such a run the cells are initially grown batchwise in a rich medium containing 5 g/l glucose. After eight hours of growth a continuous feed containing concentrated glucose, casamino acids, yeast nitrogen base and vitamins and minerals is started. The feed rate is then successively increased at eight hour intervals.

In a standard run (designated 176-3) a batch medium contained: 6 g/l of glucose; 2 g/l of $KH_2PO_4$; 2 g/l of $(NH_4)_2PO_4$; 2 g/l of casamino acids; 2 g/l of yeast nitrogen base; 2 g/l of $MgSO_4$; 0.3 ml of thiamine (1% solution) per liter; 1 ml of trace metals solution A (27 g/l of $FeCl_3$ .$6H_2O$; 2.0 g/l of $ZnCl_2$ .$4H_2O$; 2.0 g/l of $CaCl_2$ .$6H_2O$; 20 g/l of $NaMoO_4$.$2H_2O$; 1.0 g/l of $CaCl_2$.$2H_2O$; 1.9 g/l of $CaSO_4$.$5H_2O$; 0.5 g/l of $H_3BO_3$; and 100 ml/l of concentrated HCl] per liter; and 1 ml of vitamin solution A [0.42 g/l of riboflavin; 5.4 g/l of pantothenic acid; 6.1 g/l of niacin; 1.4 g/l of pyridoxine; 0.06 g/l of biotin; and 0.04 g/l folic acid] per liter. A feed medium for a standard run contained: 400 g/l of glucose; 5 g/l of $KH_2PO_4$; 5 g/l of $(NH_4)_2PO_4$; 100 g/l

of casamino acids; 12 g/l of yeast nitrogen base; 5 g/l of $MgSO_4$; 0.3 ml of thiamine (1% solution) per liter; 1 ml trace metals solution A per liter; and 1 ml vitamin solution A per liter.

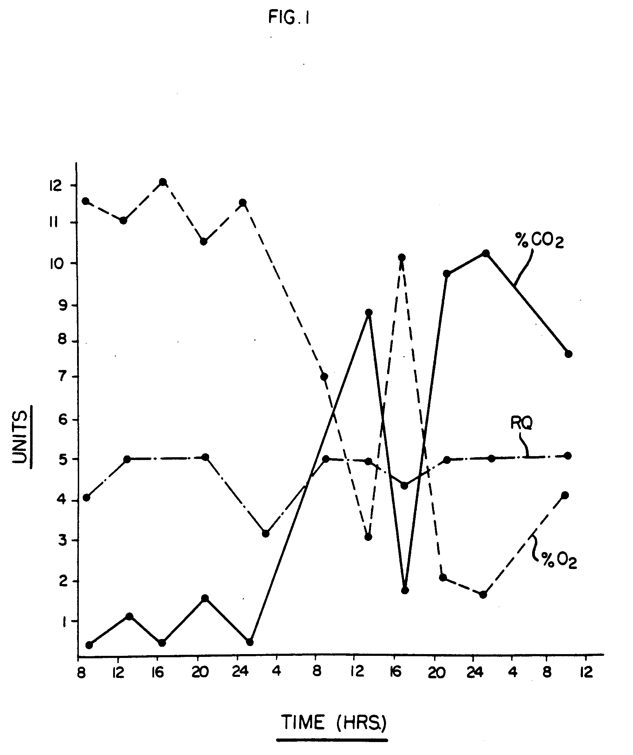

During run 176-3, the exhaust gas from the fermentor was monitored for $CO_2$ and $O_2$ using a mass spectrometer, and these values were recorded every four hours. This data is graphically depicted in Fig. 1. In addition, samples were collected and centrifuged every four hours and the culture supernatant frozen for later analysis.

Glucose analysis of the culture broth indicated that throughout the fermentation glucose was limiting, i.e., the concentration was less than 100 mg/liter. Therefore, a carbon balance between glucose and $CO_2$ plus cells was performed to determine whether all of the glucose could be accounted for. A balance over the entire time course of the fermentation was performed by graphically integrating the $CO_2$ data in Fig. 1. This integration showed that 11.15 moles of $CO_2$ were produced during the course of the fermentation. At the end of the fermentation, a total of 1630 g of glucose had been consumed and 177g of yeast had been produced. Assuming the yeast to be 50% carbon the balance works out as follows:

1630 g glucose --> 491 g $CO_2$ + 177g yeast

or on a carbon basis:

652 g C --> 134 g C + 88.5 g C

In other words, 652 - (134 + 88.5) = 429 g carbon or 66% of the glucose is not accounted for. Extracellular secreted protein was eliminated as the situs of the carbon deficit because Biuret protein assays of the culture supernatant showed only trace amounts of protein in the culture supernatant.

Glucose may be metabolized by yeast in either of two ways. Complete oxidation of glucose by the oxidative pathway produces large amounts of energy (16-28 moles of ATP/mole of glucose) during the reaction

(1)    $C_6H_{12}O_6$ --> $6CO_2$ + 6 $H_2O$ + cells

and is characterized by a RQ [respiratory quotient = moles $CO_2$ produced/moles $O_2$ consumed] of 1.0-1.2. On the other hand, partial oxidation through the fermentative pathway produces low energy yields (2 moles of ATP/mole of glucose) according to the reaction

(2)    $C_6H_{12}O_6$ --> $2C_2H_5OH$ + $2CO_2$ + cells

with an RQ>>1.0.

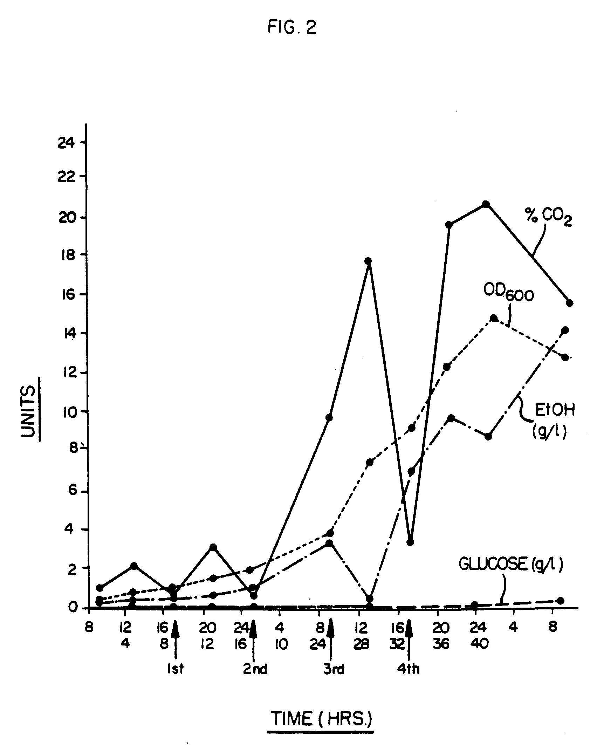

Examination of the RQ data in Fig. 1 shows the value of RQ to consistently be above 3.0. Therefore, substantial fermentative metabolism was occurring. This was verified by measuring the ethanol concentration in the broth. The profiles of ethanol, glucose, $CO_2$ and $OD_{600}$ for run 176.3 are plotted in Fig. 2. At the end of the fermentation 73 g/l of ethanol had accumulated in the medium, accounting for a total of 381 g of carbon. Including this value into the carbon balance:

(3)    glucose --> $CO_2$ + yeast + ethanol

652 g C --> 134 gC + 88.5 gC + 381 g C results in a carbon recovery of 92%. The remaining carbon deficit is most likely due to the loss of ethanol in the exhaust air or to measurement error.

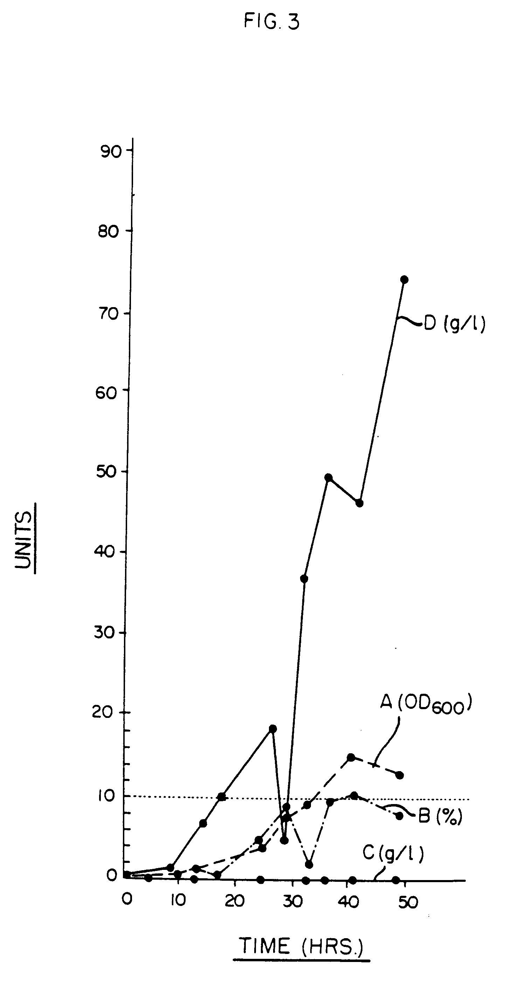

The effect of this transition from oxidative to fermentative pathways may also be seen in the growth yield data (g yeast dry weight/g glucose) for run 176-3 as reported in Table I and in Fig. 3, wherein the $OD_{600}$ (optical density at 600 nm) is labelled A, the percentage of $CO_2$ is labelled B, the concentration of glucose (in g/l) is labelled C and the concentration of ethanol (in g/l) is labelled D.

6

TABLE I

| Hours | OD 600 | Respiratory Quotient | Ethanol (g/l) | Glucose (g/l) | % $CO_2$ |
|---|---|---|---|---|---|
| 1 | 0.30 | 4 | 0.50 | 0 | 0.50 |
| 5 | 0.40 | 5 | 2 | 0 | 1 |
| 9 | 0.80 | 5 | 3 | 0 | 0.40 |
| 13 | 1.50 | 3 | 6 | 0 | 1.50 |
| 17 | 2 | 5 | 11 | 0 | 0.40 |
| 25 | 4 | 5 | 18 | 0 | 5 |
| 29 | 7.50 | 4.30 | 2 | 0 | 8.80 |
| 33 | 9.30 | 5 | 36 | 0 | 1.70 |
| 37 | 12.50 | 5 | 49 | 0 | 9.80 |
| 41 | 15 | 5 | 45 | 0.10 | 10.50 |
| 49 | 13 | 5 | 73 | 0.25 | 8 |

A drop in growth yield with time is expected because while oxidative metabolism of glucose provides 16 to 28 moles of ATP/mole of glucose, the fermentative metabolism that predominates later in fermentation provides only about one tenth as much energy or 2 moles of ATP/mole glucose.

Thus, examination of the respiratory quotient, growth yield and ethanol concentration data indicate substantial fermentative metabolism of glucose to ethanol during the fed-batch growth of S. cerevisiae. This type of metabolism results in low energy yield from glucose and hence low yield of yeast cells. The ethanol produced in turn results in a lowered maximum specific growth rate. Ethanol is a known noncompetitive growth inhibitor of yeast and obeys the equation:

(4)     $\mu_{mi} = \mu_m(K_i)/(i + K_i)$

where $\mu_{mi}$ is the reduced maximum specific growth rate that occurs when the growth inhibitor is present at a concentration of i with an inhibition constant of $K_i$. The value of $K_i$ for ethanol affecting the growth of S. cerevisiae has been reported to be about 25 grams of ethanol/liter. Therefore, as more and more ethanol is produced, the maximum specific growth rate is further and further decreased, despite the addition of glucose at increasing rates. The only course of action open to the yeast cell is the production of more ethanol because its maximum specific growth rate is lower than the addition rate of glucose.

Partial oxidation of glucose to ethanol by yeasts occurs under any of three conditions (1) at low dissolved oxygen concentrations (2) under conditions of excess glucose or (3) at too high a specific growth rate. In Example 1 there was (1) enough oxygen and (2) the glucose concentration was maintained near

7

zero; however, (3) the growth rate of the cell was maintained at its maximum because glucose was added at a rate greatly in excess of the cells maximum specific growth rate. In this light, Example 2 describes experiments wherein the specific growth rate of the culture is maintained at a lower level to provide (1) a much higher growth yield of cells from glucose and (2) a much higher final cell concentration because inhibitory concentrations of ethanol were not formed.

Example 2

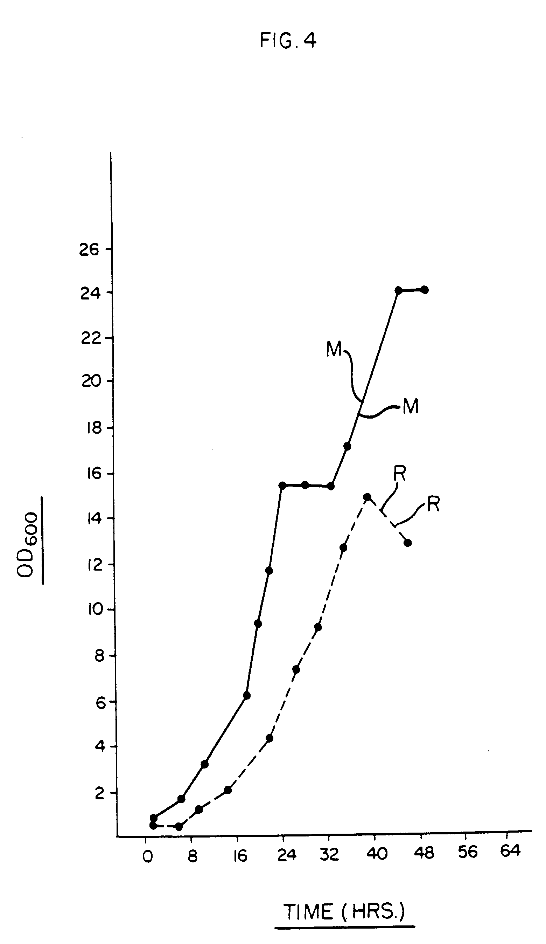

In a study of medium composition and feed rate, HBsAg-expressing cells of a strain SC RH 218/pGPD-1 [the construction of which is described in Bitter, et al., Gene, 32, 263-274 (1984)] were grown at 30°C., pH 4.5 in a run (designated 193-2) on minimal medium and in a run (designated 176-3 and described in Example 1) on a rich medium supplemented with acid-hydrolyzed casamino acids. The results are illustrated in Fig. 4 wherein a graph of $OD_{600}$ for cells grown on the minimal medium is labelled M and a graph of $OD_{600}$ for cells grown on the rich medium (as described for run 176-3) is labelled R.

In a minimal batch medium the following components were included: 5 g/l glucose; 5 g/l $K_2HPO_4$; 2 g/l $(NH_4)_2SO_4$; 1 ml of 1 M $MgSO_4$ per liter; 0.2 ml of 1% thiamine solution per liter; 1 ml of trace metals solution A per liter; 1 ml vitamin solution A per liter; 0.02 g/l inositol; and 0.17 g/l DOW® P2000 (polypropylene glycol with an average molecular weight of about 2000). In a minimal feed medium the following components were included; 400 g/l glucose; 25 g/l $KH_2PO_4$; 64 g/l $(NH_4)_2SO_4$; 15 ml of 1M $MgSO_4$ per liter; 0.5 ml of 1% thiamine solution per liter; 0.5 ml of trace metals solution A per liter; 0.5 ml vitamin solution A per liter; and 0.05 g/l inositol.

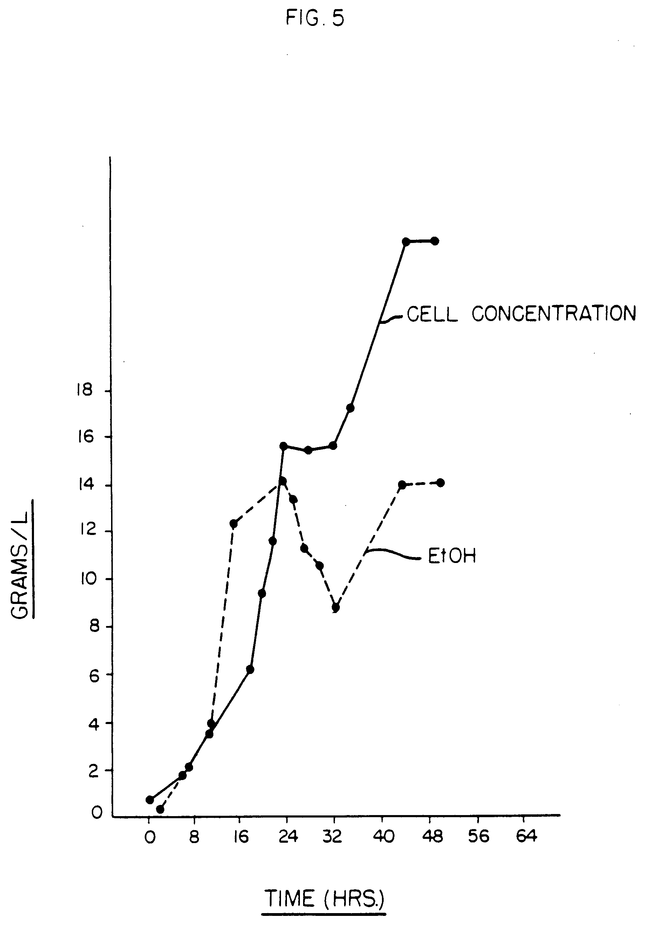

Growth on minimal medium produced a cell concentration of an OD = 24 (26 g cell dry weight/liter). At 24 hours, the addition of glucose to the fermentor was stopped. From 24 to 32 hours, the ethanol concentration decreased from 14 to 9 g/liter, but the cell concentration did not increase. Thus, the cells grown on the minimal medium did not effectively utilize accumulated ethanol in the medium for cell biosynthesis as can be derived from Fig. 5.

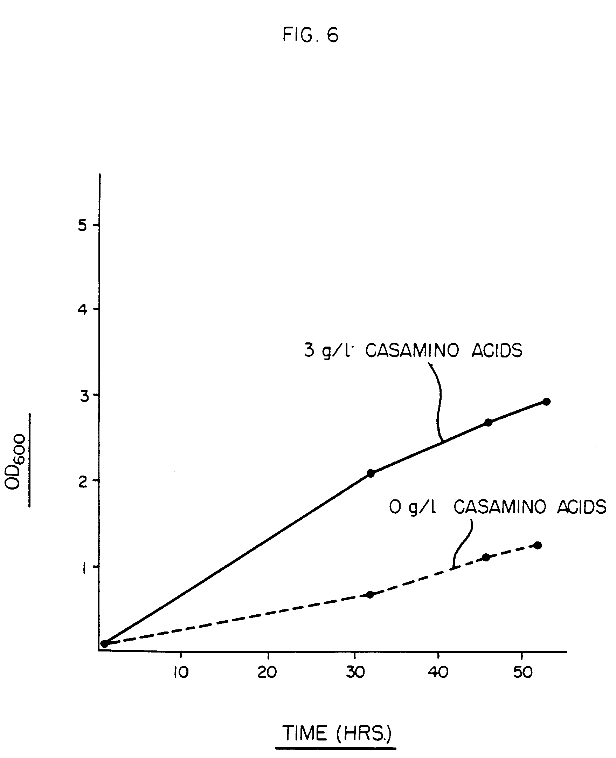

Because this strain exhibits a diauxic, i.e., a two-phase growth on glucose and ethanol in YMS, shaker flask experiments were performed to determine whether the cells would grow to higher cell concentration by growing on the produced ethanol in a medium lightly supplemented with acid hydrolyzed casamino acids. YMS medium consists of: 6.7 g/l of yeast nitrogen base without amino acids (Difco, Detroit, Michigan 48232), 5 g/l casamino acids (BBL, BBL Microbiology Systems, Becton Dickinson and Co., Cockeysville, MD 21030) and 20 g/l glucose. The results of this experiment done with duplicate flasks with a minimal medium [100 mM $(PO_4)^{-2}$; 1 g/l $NH_4Cl$; 0.5 g/l NaCl; 0.002M $MgSO_4$; 0.0002M $CaCl_2$; and 5 g/l glucose] plus 3 g/l casamino acids appear in Fig. 6. As illustrated in Fig. 6, much faster growth is obtained on ethanol when casamino acids are included in the medium.

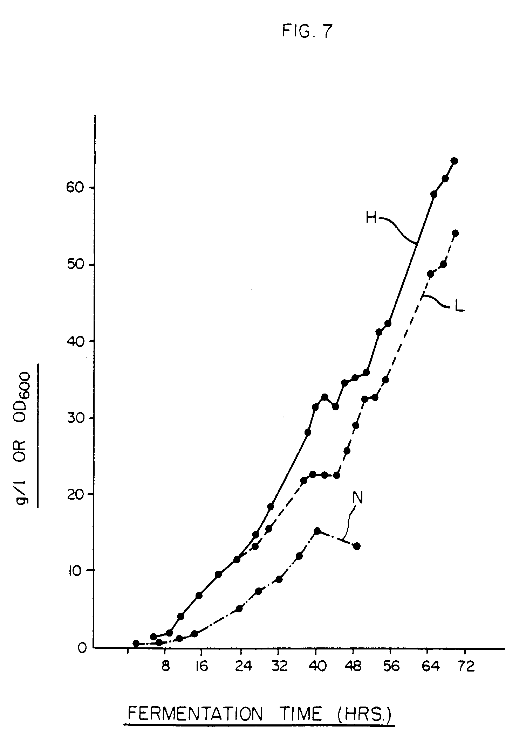

The variation of cell concentration with time for growth at 30°C. and at pH 4.5 in the presence of concentrations of 5 and 10 g/l of casamino acids included in the batch growth medium is illustrated in Fig. 7. The results of a run (designated 211-6) having 10 g/l casamino acids in the batch medium and 20 g/l of casamino acids in the feed medium is labelled H and a graph of the results of the run (designated 211-5) having 5 g/l casamino acids in the batch medium and 10 g/l of casamino acids in the feed medium is labelled L in Fig. 7. The ethanol concentration in runs 211-5 and 211-6 never rose above 2 g ethanol/liter while the ethanol concentration for run 176-3 in rich medium increased to above 70 g ethanol/liter at the end of the fermentation. A plot of the cell concentration with time for run 176-3 is included in Fig. 5 as the line labelled N.

A batch medium for run 211-5 included: 2 g/l glucose; 5 g/l casamino acids; 5 g/l $KH_2PO_4$; 2 g/l $(NH_4)_2SO_4$; 1 ml 1M $MgSO_4$ per liter; 0.2 ml 1% thiamine per liter; 1 ml trace metals solution A per liter; 1 ml vitamin solution A per liter; and 0.117 ml Dow® P2000 per liter. A feed medium for run 211-5 included 275 g/l glucose; 10 g/l casamino acids; 25 g/l $KH_2PO_4$; 50 g/l $(NH_4)_2SO_4$; 15 ml 1M $MgSO_4$ per liter; 1 ml 1% thiamine per liter; 2.5 ml trace metals solution A per liter; and 2.5 ml vitamin solution A per liter. A batch medium for run 211-6 included: 2 g/l glucose; 10 g/l casamino acids; 5 g/l $KH_2PO_4$; 2 g/l $(NH_4)_2SO_4$; 1 ml 1M $MgSO_4$; 1 ml 1M $MgSO_4$ per liter; 0.2 ml 1% thiamine per liter; 1 ml trace metals solution A per liter; 1 ml vitamin solution A per liter; and 0.17 ml Dow® P2000 per liter. A feed medium for run 211-6 included: 275 g/l glucose; 20 g/l casamino acids; 25 g/l $KH_2PO_4$; 50 g/l $(NH_4)_2SO_4$; 15 ml 1M $MgSO_4$ per liter; 1 ml 1% thiamine per liter; 2.5 ml trace metals solution A per liter; and 2.5 ml vitamin solution A per liter.

The specific growth rate of the organism must be maintained below a certain level to avoid the toxic accumulation of ethanol. As noted above, in run 176-3, ethanol accumulated to greater than 70 g/liter, thereby (1) reducing the growth yield substantially (to 0.12 g cell dry weight/g glucose) and (2) inhibiting the growth rate and final cell concentration (to 16 g cell dry weight/liter). However, in run 211-6, the growth rate was carefully controlled by the addition rate of glucose and no ethanol accumulated in the medium resulting in (1) an increased growth yield of 0.34 g cell dry weight/g glucose and (2) an increased cell concentration (to 68.2 g cell dry weight/liter).

In order to avoid ethanol accumulation in the runs 211-6 and 211-5, glucose was fed very slowly into the fermentor. It is preferable that the feed rate be increased to a point just below the point where ethanol begins to accumulate in order to allow for better fermentor productivity. This may be accomplished empirically by monitoring ethanol and respiratory quotient (RQ). As an alternative, computer-coupled control of the glucose addition rate to the RQ may be used as disclosed in Woehrer, et al., Biotechnol. Bioeng., 23, 567-581 (1981).

Example 3 describes the growth of S. cerevisiae (RH 218) strain SC RH 218/pGPD-1 containing the plasmid pGPD-1, as described in Bitter, et al., Gene, 32, 263-274 (1984), to cell concentrations of over 200 g dry weight/liter (equivalent to an $OD_{600}$ of 190 and 460 measured on a Spectronic 20 [Bausche & Lombe, Rochester, New York] or on a Beckman spectrophotometer respectively), and at expression levels up to 1.8 mg HBsAg/g dry weight cells (equivalent to 600 ng HBsAg/OD-ml) in the presence of high levels of casamino acids.

Example 3

In both of the runs on casamino acids in Example 2, there was "dead time" at t = 40 hours and at t = 50 hours where the culture was unintentionally starved for magnesium and was not growing. The culture did not grow until additional $MgSO_4$ was added. Magnesium starvation occurred because the medium was designed for much less efficient use of glucose, i.e., a growth yield of 0.15 g cell dry weight/g glucose. When the medium was balanced and the actual growth yield was much greater, i.e., 0.34 g. cell dry weight/g glucose, other nutrients became limiting. A new medium designed to take the new growth yield into account avoids this inadvertent nutrient depletion. A batch medium composition is listed in Table II.

Table II

| | |
|---|---|
| Purified Water | 7 l |
| Acid hydrolyzed casamino acids | 202 g |
| $KH_2PO_4$ | 108 g |
| $(NH_4)_2SO_4$ | 30 g |
| Glucose | 16 g |
| Inositol | 0.16 g |
| $MgSO_4$ (1M) | 32 ml |
| Trace Metals soln. A | 24 ml |
| Dow® P2000 Antifoam | 1 ml |
| Thiamine soln. (1%) | 3.2 ml |
| Vitamin soln. A | 24 ml |
| $NH_4OH$ (30% w/w as $NH_3$) and $H_3PO_4$ (85% w/w) were added as needed to maintain pH at 4.5 | |

The glucose, inositol and $MgSO_4$ are combined in purified water adjusted to 200 ml and autoclaved for about 30 minutes at 121°C. Trace metals solution A, the 1% thiamine solution and vitamin solution A are then aseptically added to form Solution Ia to about 7.0 liters of purified water in a fermentor, the casamino acids, $KH_2PO_4$, $(NH_4)_2SO_4$ and antifoaming agent are added. The fermentor is first sterilized and then cooled to about 30°C. Next Solution Ia is added to the fermentor.

The feed medium composition is shown in Table III.

Table III

| Purified Water | up to 6 l |
| Glucose | 3200 g |
| Casamino acids | 656 g |
| $KH_2PO_4$ | 17.1 g |
| $(NH_4)_2SO_4$ | 30 g |
| $MgSO_4 7H_2O$ (1M) | 90 ml |
| Thiamine Soln ($\overline{1}\%$) | 8.6 ml |
| Trace metals Soln. A | 40.5 ml |
| Vitamin Soln. A | 40.5 ml |
| Inositol | 0 .2 g |

The glucose, inositol and $MgSO_4$ are combined in purified water up to 4 liters to form Solution Ib and autoclaved at about 121°C for about 30 minutes. The casamino acids, $KH_2PO_4$ and $(NH_4)_2SO_4$ are combined in purified water up to 2 liters to form Solution IIb which is autoclaved at about 121°C for about 30 minutes. Solutions Ib and IIb are cooled to room temperature and then aseptically combined. The thiamine solution, trace metals solution A and vitamin solution A are then added to the mixture of Solutions Ib and IIb.

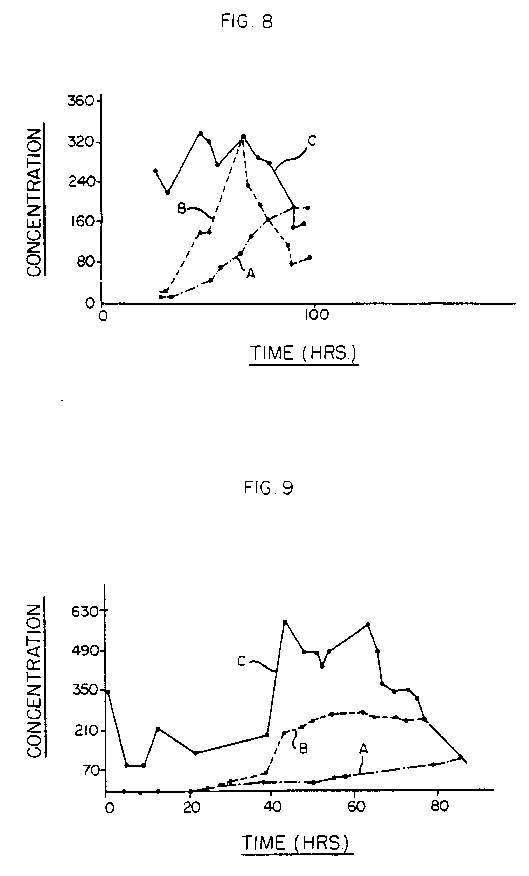

The new media were used to grow SC RH 218/pGPD-I to high cell concentrations under the glucose-feed conditions of Example 2. The implementation in a run designated 226-4 of this medium, a staged feeding schedule and manipulation of agitation, aeration and back pressure (to permit sufficient oxygen transfer into the fermentation broth) resulted in cell concentrations of greater than 190 as measured on a Spectronic 20 spectrophotometer at 600 nm. Fig. 8 depicts profiles for run 226-4 (at 30°C., pH 4.5) of cell concentration labelled A ($OD_{600}$), HBsAg concentration in mg/liter of broth (X10) labelled B and the specific HBsAg concentration (RIA Ausria from Abbott Labs in ng/OD-ml where 600 ng HBsAg/OD-ml = 1.8 mg HBsAg/g dry weight cells) labelled C.

A batch medium for run 226-4 included: 2 g/l glucose; 5 g/l casamino acids; 13.2 g/l $KH_2PO_4$; 10 g/l $(NH_4)_2SO_4$; 8 ml 1M $MgSO_4$ per liter; 0.4 ml 1% thiamine per liter; 3 ml trace metals solution A per liter; 3 ml vitamin solution A per liter; and 0.02 g/l inositol. A feed medium for run 226-4 included: 528.5 g/l glucose; 2.86 g/l $KH_2PO_4$; .50 g/l $(NH_4)_2SO_4$; 15 ml 1M $MgSO_4$ per liter; 1.43 ml 1% thiamine per liter; 7.14 ml trace metals solution A per liter; 7.14 ml vitamin solution A per liter; and 0.03 g/l inositol.

In Fig. 8, the concentration of HBsAg peaks at 33 mg HBsAg/liter approximately 70 hours into the fermentation (at an OD of 120). After this point, the HBsAg concentration rapidly and continually drops until the end of the fermentation, despite the further increase in cell concentration to an OD of about 190.

Western blots confirmed a decrease in the specific amount of antigen protein during this time interval, and thus the decrease in RIA HBsAg particle concentration may be attributed at least partly to the decrease in the proteinaceous component. It is not clear, however, whether the antigen protein is undergoing proteolysis and/or is simply not being synthesized by the cell and the amount made early in the fermentation diluted by the growth of cells with little or no antigen protein.

Proteolysis is normally greatest in cells that are growing slowly or are in stationary phase or that are grown in minimal medium or that are grown at super optimal temperatures. Because the cells in run 226-4 were grown in minimal medium only lightly supplemented with casamino acids, the initial approach taken was to enrich the growth medium with casamino acids. In run 211-5 of Example 2, the batch and feed medium were similar to that used in run 226-4 except that additions of casamino acids of 5 g/liter and 10 g/liter were made shotwise at approximately 38 and 54 hours respectively.

As shown in Fig. 9, in which $OD_{600}$ is labelled A, the addition of casamino acids in a run designated 216-6 not only helped in "stabilizing" HBsAg [note the plateau in (mg HBsAg/liter) X 10 labelled B] from 55 to 75 hours) but also caused the specific amount of antigen (in ng/OD-ml) [labelled C] to jump dramatically at 38 and 55 hours. Whether the casamino acids reduced the proteolytic loss of HBsAg or caused a stimulation in HBsAg, the net result was an increase in amounts of HBsAg.

A batch medium for run 216-6 included: 2 g/l glucose; 5 g/l casamino acids; 13.2 g/l $KH_2PO_4$; 10 g/l $(NH_4)_2SO_4$; 4 ml 1M $MgSO_4$ per liter; 0.4 ml 1% thiamine per liter; 1 ml trace metals solution A per liter; 1 ml vitamin solution A per liter; 0.04 g/l filter-sterilized inositol; and 0.2 ml Dow® P2000 per liter. A feed medium for run 216-6 included: 528.57 g/l glucose; 1.86 g/l $KH_2PO_4$; 50 g/l $(NH_4)_2SO_4$; 15 ml 1M $MgSO_4$ per liter; 1.43 ml 1% thiamine per liter; 2.86 ml trace metals solution A per liter; and 2.86 ml vitamin solution A per liter.

One further possibility for the HBsAg decrease was due to plasmid loss and/or mutation of the culture while in the fermentor. To test this hypothesis, samples were taken every 4-8 hours during the fermentation and plated onto non-selective YPG medium (10 g/l of yeast extract (available from BBL, Cockeysville, Maryland), 20 g/l of bacto-tryptone (available from BBL, Cockeysville, Maryland), and 20 g/l of glucose) and then replica-plated onto selective YMS medium. For all time points of the fermentation sampled, the plasmid stability was greater than 90%. Twenty colonies were then picked at random from a time point late in the fermentation where the HBsAg was decreasing. These colonies were then used to innoculate 20 flasks of YMS medium and grown for 48 hours on a rotary shaker. The cultures in every flask produced HBsAg with expression levels of 100-200 ng HBsAg/OD-ml. Therefore, culture instability was ruled out as the cause of the decrease in HBsAg.

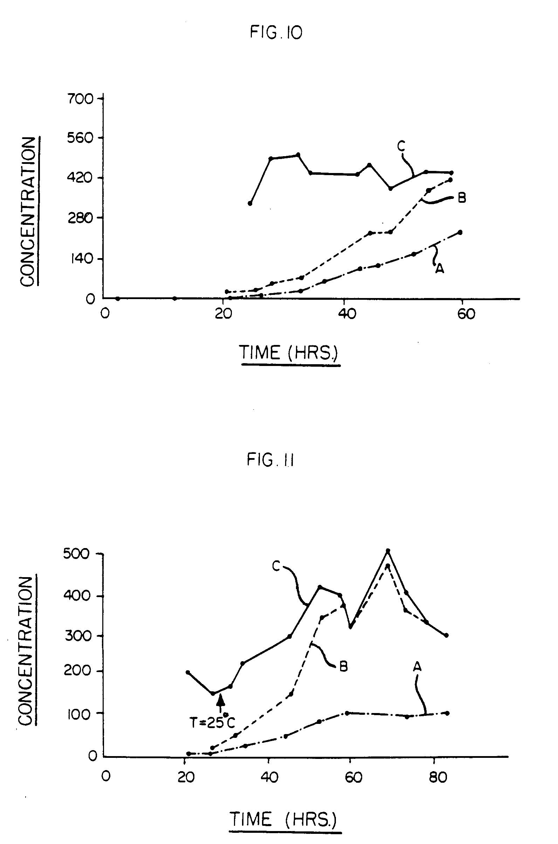

Due to the increased levels of HBsAg caused by the shotwise addition of casamino acids, the next fermentation, designated run 248-4 incorporated increased levels of casamino acids in both the batch medium (20 g/l as opposed to 5 g/l in run 211-6 and run 176-3 and in the feed (110 g/l as opposed to 0 g/l in runs 211-6 and 176-3. The profiles (at 30°C.,pH 4.5) of $OD_{600}$ in a plot labelled A, mg HBsAg/liter, in a plot labelled B, and ng HBsAg/OD-ml, in a plot labelled C, for run 248-4 are shown in Fig. 10. The expression level of HBsAg is seen to remain constant at approximately 200 ng HBsAg/OD-ml throughout the course of the fermentation. The increased stability of HBsAg production in run 248-4 was desirable. However, the expression level of 200 ng HBsAg/OD-ml was 2-3 times lower than in run 211-6.

A batch medium for run 248-4 included: 2 g/l glucose; 25.25 g/l casamino acids; 13.5 g/l $KH_2PO_4$; 3.75 g/l $(NH_4)_2SO_4$; 4 ml 1M $MgSO_4$ per liter; 0.4 ml 1% thiamine per liter; 3 ml trace metals solution A per liter; 3 ml vitamin solution A per liter; 0.02 g/l inositol; and 0.13 ml Dow® P2000 per liter. A feed medium for run 248-4 included: 532.5 g/l glucose; 209.25 g/l casamine acids; 2.85 g/l $KH_2PO_4$; 5 g/l $(NH_4)_2SO_4$; 15 ml 1M $MgSO_4$ per liter; 6.75 ml trace metals solution A per liter; 6.75 ml vitamin solution A per liter; 0.02 g/l inositol; and 0.13 ml Dow® P2000 per liter.

In Example 3, the addition of casamino acids in both a pulsatile and a continuous feed helped in stabilizing the formation of the HBsAg particle, although this stabilization in the continuous feed was apparently at the expense of reduced expression level. In order to minimize the action of proteolytic enzymes, growth at reduced temperatures, is employed in Example 4.

Example 4

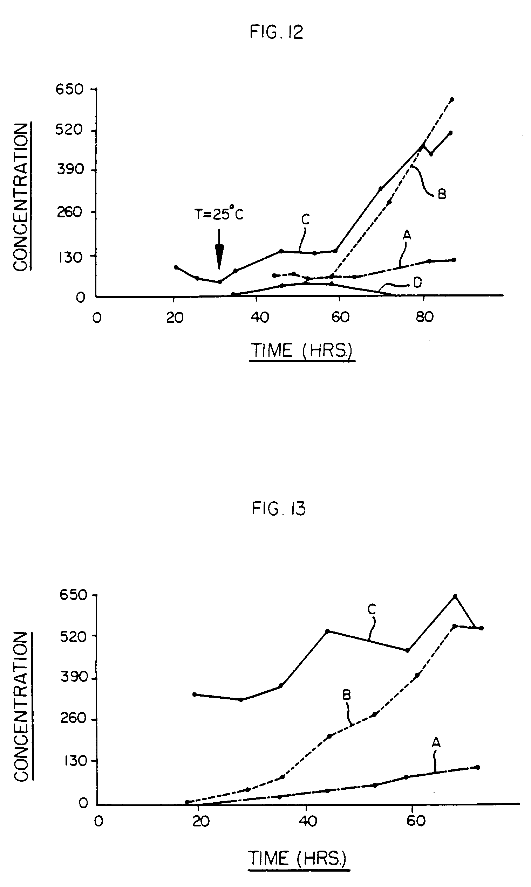

In a run designated 262-4, the strain SC RH 218/pGPD-I was grown in a medium enriched with casamino acids (about 25 g/liter) and fed a medium which was also enriched with the batch casamino acids (about 110 g/liter). (The batch and feed media were otherwise identical with those of run 248-4.) The temperature in this run was decreased from the 30°C. (pH 4.5) normally used to 25°C at 28 hours into the fermentation. The results of this run are tabulated in Table IV and graphed in Fig. 11 wherein a plot of $OD_{600}$ is labelled A, wherein a plot of (mg/l) X 10 is labelled B and wherein a plot of ng/OD-ml is labelled C.

The expression level peaked at 489 ng/OD-ml at t = 70 hours or with 96 OD units this gives 46.9 mg HBsAg/liter. At approximately t = 63 hours the feed medium was depleted. However, both the expression level and the total amount of HBsAg continued to increase after this point up until 70 hours at which time the amount of HBsAg decreased sharply, presumably due to the action of proteases which were induced after cessation of growth.

Table IV

| Time (hr.) | $OD_{600}$ | NG/OD-ML | MG/L X10 |
|---|---|---|---|
| 21 | 3.90 | 206 | 8 |
| 27 | 11.60 | 147 | 17 |
| 31 | 22.30 | 166 | 37 |
| 35 | 26 | 223 | 58 |
| 46 | 50 | 297 | 144 |
| 54 | 82 | 414 | 339 |
| 58 | 92 | 397 | 365 |
| 60 | 100 | 320 | 320 |
| 70 | 96 | 489 | 469 |
| 73 | 89 | 405 | 360 |
| 80 | 103 | 320 | 330 |
| 84 | 100 | 287 | 287 |

The medium used in a run designated 262-5 was a close-to-minimal medium with only 5 g/liter of CAA added to the initial batch medium. Ammonium sulfate served as the sole nitrogen source in the feed.

A batch medium for run 262-5 included: 2 g/l glucose; 5 g/l casamino acids; 13.5 g/l $KH_2PO_4$; 10 g/l $(NH_4)_2SO_4$; 4 ml 1M $MgSO_4$ per liter; 0.4 ml 1% thiamine per liter; 3 ml trace metals solution A per liter; 3 ml vitamin solution A per liter; 0.02 g/l inositol; and 0.13 ml Dow® P2000 per liter. A feed medium for run 262-5 included: 532.5 g/l glucose; 2.85 g/l $KH_2PO_4$; 50 g/l $(NH_4)_2SO_4$; 15 ml 1M $MgSO_4$ per liter; 1.43 ml 1% thiamine per liter; 6.75 ml trace metals solution A per liter; 6.75 ml vitamin solution A per liter; and 0.04 g/l inositol.

As in run 262-4, the temperature was decreased from 30 to 25°C at t = 28 hours.

The results of run 262-5 (at pH 4.5) appear in Table V and in Fig. 12 wherein a plot of $OD_{600}$ is labelled A, wherein a plot of (mg/l) X 10 if labelled B, wherein a plot of ng/OD-ml is labelled C and wherein a plot of (g/l of ethanol) X 10 is labelled D. After lowering the temperature to 25°C. from 30°C., the expression level increased and continued increasing until termination of the fermentation. The total amount of HBsAg also increased until the end of the fermentation reaching a maximum of 61.7 mg HBsAg/liter.

### Table V

| Time (hr.) | OD$_{600}$ | NG/OD-ML | (MG/L) X10 | EtOH (G/L) X10 |
|---|---|---|---|---|
| 21 | 3.20 | 100 | 3 | |
| 27 | 8.20 | 68 | 5 | |
| 31 | 16.30 | 51 | 8 | |
| 35 | 13 | 89 | 11 | |
| 46 | 27 | 145 | 39 | 74.60 |
| 48 | | | 83.10 | |
| 50 | | | 72.40 | |
| 52 | 44 | 140 | 50 | |
| 53 | 51 | 149 | 62 | |
| 59 | 80 | 369 | 76 | |
| 72 | | | 295 | 3.10 |
| 78 | | | 3.60 | |
| 80 | 103 | 478 | 493 | |
| 82 | 114 | 455 | 518 | |
| 86 | 118 | 523 | 617 | |

In a run designated 260-4 (at pH 4.5) approximately the same enriched medium and feed used in run 248-4 were employed but a reduced temperature of 25°C was used starting at time zero. The results of run 260-4 appear in Table VI and are illustrated in Fig. 13 wherein a plot of OD$_{600}$ is labelled A, wherein a plot of (mg/l) X 10 is labelled B and wherein a plot of ng/OD-ml is labelled C.

### Table VI

| Time (hr.) | OD$_{600}$ | NG/OD-ML | MG/L X10 |
|---|---|---|---|
| 19.50 | 4 | 336 | 13.40 |
| 27.50 | 11.30 | 320 | 36.10 |
| 35.50 | 22.70 | 360 | 81.70 |
| 44 | 37 | 533 | 197 |
| 48.50 | 45 | 509 | 229 |
| 53.50 | 54 | 490 | 265 |
| 59.50 | 75 | 473 | 355 |
| 68.00 | 86 | 636 | 547 |
| 72.50 | 103 | 522 | 538 |

Time seen in Figs. 11 and 12. However, a slight unexplained decrease in HBsAg occurred from 54.7 mg/liter at 68.5 hours to 53.8 mg/liter at 72.5 hours.

Thus, reduced temperature of 25°C appears to stabilize HBsAg production, possibly by reducing the concentration and/or activity of proteolytic enzymes. HBsAg levels of over 60 mg/liter have been obtained

13

EP 0 233 937 B1

by reducing the temperature in both media enriched with casamino acids (61.7 mg/liter) and non-enriched (63.6 mg/liter. At 20 μg HBsAg/dose of vaccine, these numbers represent over 3000 doses/liter of fermentation broth.

Although the present invention has been described in terms of preferred embodiments, it is understood that modifications and improvements will occur to those skilled in the art. Consequently, it is intended that the present invention include all such variations which come within the scope of the invention as claimed.

## Claims

1. A medium for culturing yeast capable of expressing a foreign gene product comprising:

    balanced free amino acids at a combined total concentration between about 25 g/l and about 160 g/l.

2. The medium according to Claim 1 wherein the balanced free amino acids are casamino acids.

3. The medium according to Claim 2 wherein the combined total concentration of balanced free amino acids is about 110 g/l.

4. A method for the fermentation of yeast capable of expressing an exogenous gene product comprising the steps of:

    introducing the yeast into a culture medium comprising balanced free amino acids at a combined total concentration between about 25 g/l and about 160 g/l; and
    culturing the yeast at a temperature of about 25 degrees C.

5. The method according to Claim 4 wherein said introducing step comprises the step of maintaining a carbohydrate-derived carbon source concentration in the culture medium at substantially zero so that ethanol production by the yeast is minimized.

6. The method according to Claim 5 further comprising the step of saturating the culture medium with oxygen at least 30% of air saturation.

7. A method for the fermentation of yeast capable of expressing an exogenous gene product comprising the steps of:

    culturing the yeast in a medium having a carbohydrate-derived carbon source concentration maintained at substantially zero so that ethanol production by the yeast is minimized, providing in the medium balanced free amino acids at a concentration between about 20g/l and about 160g/l; and
    maintaining the temperature of the medium at about 25°C.

## Patentansprüche

1. Medium zum Kultivieren von Hefe, die in der Lage ist, ein fremdes Gen-Produkt zu exprimieren, welches umfaßt:

    ausgewogene freie Aminosäuren mit einer kombinierten Gesamtkonzentration zwischen etwa 25 g/l und etwa 160 g/l.

2. Medium nach Anspruch 1, dadurch gekennzeichnet, daß die ausgewogenen freien Aminosäuren Casaminosäuren sind.

3. Medium nach Anspruch 2, dadurch gekennzeichnet, daß die kombinierte Gesamtkonzentration ausgewogener freier Aminosäuren etwa 110 g/l beträgt.

4. Verfahren zur Fermentation von Hefe, die in der Lage ist, ein exogenes Gen-Produkt zu exprimieren, welches die Schritte umfaßt:

    Einbringen der Hefe in ein Kulturmedium, das ausgewogene freie Aminosäuren mit einer kombinierten Gesamtkonzentration zwischen etwa 25 g/l und etwa 160 g/l umfaßt; und

14

Kultivieren der Hefe bei einer Temperatur von etwa 25 ° C.

5. Verfahren nach Anspruch 4, dadurch gekennzeichnet, daß der Einbringschritt den Schritt umfaßt, daß die Konzentration der von Kohlehydrat abgeleiteten Kohlenstoffquelle im Kulturmedium bei im wesentlichen 0 gehalten wird, so daß die Ethanolproduktion durch die Hefe minimiert ist.

6. Verfahren nach Anspruch 5, weiter gekennzeichnet durch den Schritt, daß das Kulturmedium mit Sauerstoff zu wenigstens 30 % von Luftsättigung gesättigt wird.

7. Verfahren zur Fermentation von Hefe, die in der Lage ist, ein exogenes Gen-Produkt zu exprimieren, welches die Schritte umfaßt:

Kultivieren der Hefe in einem Medium, wobei die Konzentration einer von Kohlehydrat abgeleiteten Kohlenstoffquelle bei im wesentlichen 0 gehalten ist, so daß Ethanolproduktion durch die Hefe minimiert wird, Bereitstellen ausgewogener freier Aminosäuren mit einer Konzentration zwischen etwa 20 g/l und etwa 160 g/l im Medium; und

Halten der Temperatur des Mediums bei etwa 25 ° C.

**Revendications**

1. Milieu pour cultiver de la levure capable d'exprimer un produit génétique étranger, comprenant :
   des acides aminés libres équilibrés en une concentration combinée totale entre environ 25 g/l et environ 160 g/l.

2. Milieu selon la revendication 1, dans lequel les acides aminés libres équilibrés sont des acides casaminés

3. Milieu selon la revendication 2, dans lequel la concentration combinée totale des acides aminés libres équilibrés est d'environ 110 g/l.

4. Procédé pour la fermentation de levure capable d'exprimer un produit génétique exogène comprenant les étapes consistant à :
   - introduire la levure dans un milieu de culture comprenant des acides aminés libres équilibrés en une concentration combinée totale entre environ 25 g/l et environ 160 g/l ; et
   - cultiver la levure à une température d'environ 25 ° C.

5. Procédé selon la revendication 4, dans lequel ladite étape d'introduction comprend l'étape consistant à maintenir une concentration de source de carbone dérivée de carbohydrates dans le milieu de culture à sensiblement zéro de sorte que la production d'éthanol par la levure soit minimisée.

6. Procédé selon la revendication 5, comprenant de plus l'étape consistant à saturer le milieu de culture avec de l'oxygène à au moins 30 % de la saturation de l'air.

7. Procédé pour la fermentation de levure capable d'exprimer un produit génétique exogène comprenant les étapes consistant à :
   - cultiver la levure dans un milieu ayant une concentration de source de carbone dérivée de carbohydrates maintenue à sensiblement zéro de sorte que la production d'éthanol par la levure soit minimisée, en fournissant dans le milieu des acides aminés libres équilibrés en une concentration entre environ 20 g/l et environ 160 g/l ; et
   - maintenir la température du milieu à environ 25 ° C.

FIG. 1

FIG. 2

FIG. 3

FIG. 4

FIG. 5

FIG. 6

FIG. 7

FIG. 8

FIG. 9

FIG. 10

FIG. 11

TIME (HRS.)

FIG. 12

FIG. 13